# EUROPÄISCHE PATENTSCHRIFT

(11) **EP 1 850 102 B1**
(45) Veröffentlichungstag und Bekanntmachung des Hinweises auf die Patenterteilung: **10.02.2010**
(21) Anmeldenummer: 07106557.7
(22) Anmeldetag: 20.04.2007
(51) Int. Cl.: G01K 1/20

(54) **Messvorrichtung zum Überprüfen der Effektivität eines Dampfsterilisationsprozesses**
Measuring device for checking the effectiveness of a steam sterilisation process
Dispositif de mélange destiné à la vérification de l'efficacité d'un processus de stérilisation à la vapeur

(30) Priorität: 29.04.2006 DE 202006006926 U
(43) Veröffentlichungstag der Anmeldung: 31.10.2007
(73) Patentinhaber: ebro Electronic GmbH & Co. KG, 85055 Ingolstadt (DE)
(72) Erfinder: Klün, Wolfgang, 85049 Ingolstadt (DE)
(74) Vertreter: Schlief, Thomas P.

(56) Entgegenhaltungen:
- WO-A-93/21964
- WO-A-97/12637
- US-A- 4 115 068

## Beschreibung

Die Erfindung betrifft eine Messvorrichtung zum Überprüfen der Effektivität eines Dampfsterilisationsprozesses in einem Autoklaven durch zumindest Temperaturmessungen gemäß dem Oberbegriff des Anspruches 1.

Im Gesundheitswesen sowie in vielen anderen Industriezweigen ist es fast immer notwendig, die Effektivität von Prozessen bei der Sterilisation von Ausrüstung, wie beispielsweise medizinischen Geräten, Instrumenten, Kleidung und auch Einwegartikeln, zu überprüfen. Hierbei ist der Begriff Sterilisation allgemein definiert als Verfahren der vollständigen Zerstörung von Mikroorganismen einschließlich solcher Strukturen wie Viren, Sporen, Hefe und Pilze. Üblicherweise wird in Krankenhäusern ein Sterilitätsindikator in zu sterilisierende Artikel eingebracht. Ein derartiges Verfahren erlaubt die direkte und genaue Überprüfung des Sterilisationsprozesses.

Ein bekanntes Verfahren zur Überprüfung des Sterilisationsprozesses wird durch eine doppelte Temperaturmessung realisiert. Hierbei ist ein erster Temperatursensor direkt dem Sterilisationsraum ausgesetzt, während ein zweiter Temperatursensor innerhalb einer Messkammer angeordnet ist und die dortige Temperatur misst. Das Innere der Messkammer ist hierbei über eine kleine Öffnung mit dem Sterilisationsraum verbunden, so dass Dampf durch die Öffnung in die Messkammer eindringen kann. Aus dem Unterschied der beiden gemessenen Temperaturen sowie deren zeitlichen Verlauf kann auf die Effektivität des Sterilisationsprozesses zurückgeschlossen werden. Eine entsprechend bekannte Vorrichtung weist hierzu einen Karbonblock um den zweiten Temperatursensor auf. Dieser fest mit dem Grundkörper verbundene Karbonblock dient insbesondere zur Isolierung des zweiten Temperatursensors gegenüber dem Sterilisationsraum.

Die WO 97/12637 beschreibt eine Messvorrichtung gemäß dem Oberbegriff des Anspruchs 1. Diese Vorrichtung weist einen Innenraum auf, in dem die Elektronik sowie ein erster und ein zweiter Temperatursensor untergebracht sind. Der zweite Temperatursensor weist hierbei mit seiner Rückseite in diesen Innenraum, während seine den Messfühler tragende Vorderseite derart in einem Bohrloch eines Hohlkörpers eingeführt ist, dass er die Temperatur in diesem Bohrloch (das als Messraum dient) misst.

Die US 4,115,068 offenbart eine Vorrichtung zum Detektieren von Luft während eines Sterilisationsprozesses. Hierzu ist eine Wärmeindikatorstreifen in einem Messraum angeordnet, wobei der Messraum mit einem Pumpmechanismus evakuiert werden kann. Mittels eines Ventils kann Luft in den Messraum gelangen und aus diesem entweichen.

Es ist Aufgabe der vorliegenden Erfindung, die bekannte Vorrichtung leichter handhabbarer zu machen.

Diese Aufgabe wird durch eine Messvorrichtung mit den Merkmalen des Anspruchs 1 gelöst.

Die erfindungsgemäße Messvorrichtung zeichnet sich dadurch aus, dass die Messkammer zumindest teilweise reversibel mit dem Grundkörper verbunden ist, um sie von diesem zu trennen. Diese erfindungsgemäße Ausgestaltung bietet den Vorteil, dass an den Innenwänden der Messkammer abgesetztes Kondensationswasser oder sonstiger Schmutz in einfacher Weise entfernt werden kann. Hierzu wird die Messkammer bzw. ein Teil der Messkammer von der restlichen Messvorrichtung abgenommen und beispielsweise mit Hilfe eines Tuches vom Kondensationswasser bzw. Schmutz gereinigt. Auch ist es mittels der Erfindung möglich, die Messkammer für unterschiedliche Anwendungen auszutauschen. Andere Messkammern können - je nach Anforderung-hierbei andere geometrische Ausmessungen aufweisen und/oder aus einem anderen Material bestehen.

Die erfindungsgemäße Messkammer weist einen zumindest einseitig offenen Hohlkörper auf, der über den zweiten Temperatursensor stülpbar ist. Da die üblicherweise verwendeten Temperatursensoren langgestreckt ausgebildet sind, ist dieser Hohlkörper bevorzugt hohlzylinderförmig und länglich ausgebildet. Es ist darauf zu achten, dass das Innere der Messkammer hierbei ein für die Messgenauigkeit hinreichendes Volumen aufweist.

Erfindungsgemäß umfasst die Messkammer eine Halterung, die mit dem Grundkörper verbunden wird, während der genannte Hohlkörper vorzugsweise auf die Halterung aufsetzbar ist. Die Halterung ist entweder fest mit dem Grundkörper verbunden oder auch reversibel lösbar von diesem ausgebildet, beispielsweise versteckbar, verschraubbar und/oder verrastbar.

Gemäß dem oben Gesagten ist die Messkammer mindestens zweiteilig ausgebildet. Hierbei kann ein erster Teil fest oder ebenfalls lösbar am Grundkörper angeordnet sein (nach dem oben Gesagten entspricht dies der Halterung), während ein zweiter Teil der Messkammer mit dem ersten Teil reversibel lösbar ausgebildet ist (nach dem oben Gesagten entspricht dies dem Hohlkörper). Beim Abnehmen des zweiten Teils wird der zweite Temperatursensor vorteilhafterweise frei zugänglich.

Besonders bevorzugt ist der Hohlkörper aus einem Kunststoff gefertigt, der resistent gegen hohe Temperaturen ist, beispielsweise PEEK oder PTFE. Diese Wahl stellt sicher, dass die Messkammer unter den extremen Bedingungen in einem Dampfautoklaven auch über lange Zeit zuverlässig arbeitet.

Vorzugsweise befindet sich in dem Hohlkörper eine Durchgangsöffnung mit geeignetem Durchmesser, damit Dampf vom Sterilisationsraum in das Innere der Messkammer gelangen kann. Bei einer alternativen Ausführung ist die mindestens eine Durchgangsöffnung in einem anderen als dem hohlkammerförmigen Teil der Messkammer vorgesehen, beispielsweise in einer Halterung , auf die der Hohlkörper aufgesetzt ist (s.u.).

Bei einer diesbezüglich vorteilhaften Ausführung über- bzw. umgreift der Hohlkörper zumindest teilweise die Halterung, beispielsweise mit Hilfe einer Verrastung oder Verschraubung. Diese Ausbildung hat den Vorteil, dass der Hohlkörper schnell und unproblematisch von der Halterung gelöst werden kann, um den Hohlkörper beispielsweise von Kondensationswasser zu reinigen oder den Temperatursensor zu reparieren bzw. - falls dieser lösbar ausgebildet ist - auszuwechseln.

Bei einer diesbezüglichen Ausgestaltung ist der zweite Temperatursensor mit dem Grundkörper verbindbar, beispielsweise in eine entsprechende Aufnahme am Grundkörper einsteckbar und dort mit der Elektronik verbunden. Es bietet sich an, wenn der zweite Temperatursensor nicht nur in der Messkammer gelagert ist, sondern auch durch die genannte Halterung hin zum Grundkörper geführt ist. Hierdurch wird eine stabile Lagerung des Temperatursensors mit dem Grundkörper realisiert. Außerdem lässt sich ein Eindringen von Dampf - außer durch die genannte Durchgangsöffnung - wirksam durch entsprechende Steck- und Schraubverbindungen verhindern.

Bei einer bevorzugten Ausführungsform ist in dem Grundkörper ein elektronischer Speicher, ein sogenannter Datenlogger, vorgesehen, der in bestimmten zeitlichen Abständen die Temperaturwerte des ersten und des zweiten Temperatursensors abspeichert. Diese Werte sind beispielsweise über eine drahtlose Datenübertragung auslesbar, was während oder auch nach dem Sterilisationsprozess durchgeführt werden kann. Alternativ kann eine Datenübertragung über einen entsprechenden Abgriff am Grundkörper realisiert werden. Andere Datenübertragungsmöglichkeiten sind ebenfalls möglich.

Einer größeren Kontrollsicherheit kommt es zugute, wenn am Anfang des Sterilisationsprozesses eine Überprüfung des Vakuums vorgenommen wird. Ein derartiges Vakuum wird vorteilhafterweise erzeugt, um die Luft aus dem Autoklav zu entfernen. Üblicherweise wird hierbei durch mehrmaliges Evakuieren im Wechsel mit Dampfeinströmungen ein sog. fraktioniertes Vorvakuum erzeugt. Der Drucksensor dient hierbei zur Überprüfung des Vorvakuums. Auch kann der Drucksensor zur Verifizierung der theoretischen Dampftemperatur verwendet werden.

Vorteilhafterweise ist der genannte mindestens eine Drucksensor mit dem Grundkörper verbindbar. Dies bedeutet, dass gemäß einer bevorzugten Ausführungsform sowohl die beiden Temperatursensoren als auch der mindestens eine Drucksensor am Grundkörper angeordnet sind und über elektrische Leitungen mit der Elektronik im Grundkörper verbunden sind.

Vorteilhafte Weiterbildungen der Erfindung sind durch die Merkmale der Unteransprüche gekennzeichnet.

Im Folgenden wird die Erfindung anhand der Figuren näher erläutert. Es zeigen:
- **Figur 1**: eine Seitenansicht einer erfindungsgemäßen Messvorrichtung mit teilweise aufgebrochenem Grundkörper;
- **Figur 2**: einen Hohlkörper einer Messkammer im Längsschnitt;
- **Figur 3**: eine Halterung für den Hohlkörper gemäß der Figur 2, im Längsschnitt, und
- **Figur 4**: eine Aufsicht auf die Messvorrichtung gemäß der Figur 1.

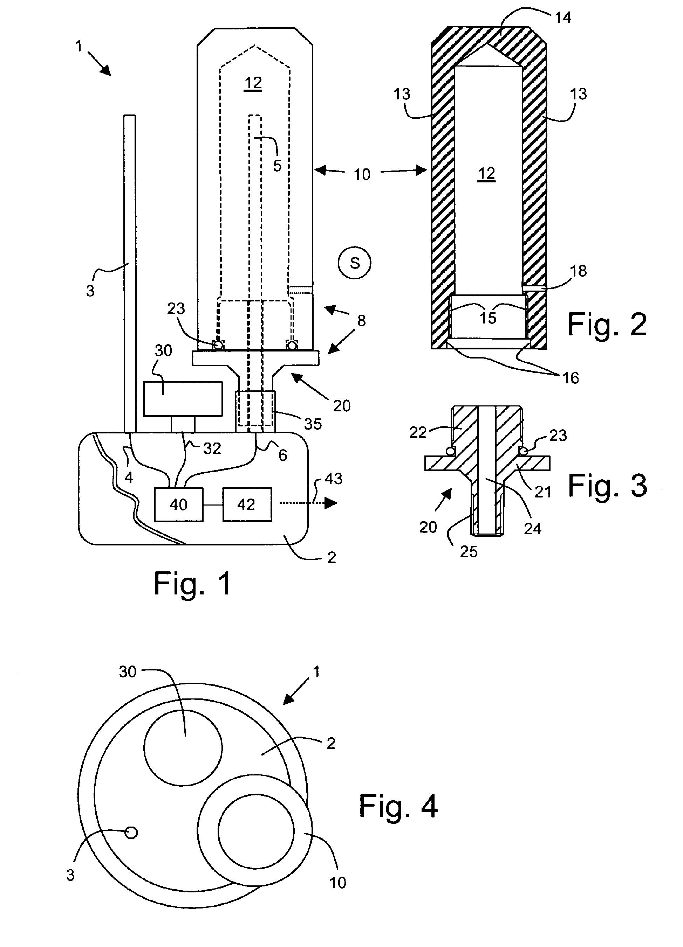

In der Figur 1 ist in der Seitenansicht und in Figur 4 in Aufsicht eine erfindungsgemäße Messvorrichtung 1 dargestellt. Diese umfasst einen zylindrischen Grundkörper 2, dessen Vorderwand in Figur 1 zum Teil entfernt dargestellt ist. Innerhalb des Grundkörpers 2 sind schematisch elektronische Einheiten 40, 42 dargestellt, auf die weiter unten näher eingegangen wird. Auf der Oberseite des Grundkörpers 2 ist ein länglicher erster Temperatursensor 3 angeordnet, beispielsweise verschweißt oder lösbar angeordnet. Weiterhin ist ein zweiter Temperatursensor 5 gleicher Ausgestaltung mit dem Grundkörper 2 verbunden, vorliegend verschweißt. Der zweite Temperatursensor 5 befindet sich innerhalb einer Messkammer 8, die vorliegend zweiteilig ausgebildet ist. Weiterhin ist an der Oberseite des Grundkörpers 2 ein Drucksensor 30 vorhanden. Die drei Sensoren 3, 5 und 30 sind über Leitungen 4, 6 bzw. 32 mit einem elektronischen Speicher 40 verbunden, der die von den Sensoren 3, 5 und 30 registrierten Temperatur- bzw. Druckwerte speichert. Diese Werte werden an eine Funkeinheit 42 übermittelt, die ihrerseits über eine Funkverbindung 43 (durch einen Pfeil symbolisiert) die Daten an eine externe, nicht dargestellte Empfangseinrichtung mit angeschlossener Auswerteeinheit übermittelt.

In den Figuren 1, 2 und 3 ist der Aufbau der Messkammer 8 genauer dargestellt. Die vorliegend beiden Teile der Messkammer 8 sind einerseits ein zylindrischer Hohlkörper 10 und andererseits eine Halterung 20, auf die der Hohlkörper 10 aufschraubbar ist. Zu diesem Zweck weist der Hohlkörper 10 einen im wesentlichen zylindrischen Hohlraum bzw. Messraum 12 auf, der den zweiten Temperatursensor 5 berührungslos umschließt. Der Kammerraum 12 wird seitlich begrenzt durch die im Querschnitt kreisringförmige Seitenwand 13 und nach oben durch die Deckenwand 14. Nach unten hin ist der Hohlkörper 10 offen, damit der zweite Temperatursensor 5 in den Messraum 12 eingeführt werden kann.

Die genannte Halterung 20 umfasst eine scheibenartige Platte 21, auf der zentrisch ein mit der Platte 21 einstückig ausgebildeter Zylinderblock 22 mit Außengewinde angeordnet ist. Im Übergangsbereich zwischen Platte 21 und Zylinderblock 22 ist eine Ringnut mit einem O-Ring 23 vorgesehen.

Hierzu korrespondierend weist der Hohlkörper 10 (Figur 2) ebenfalls eine umlaufende innere Ringnut 16 auf, in welcher der O-Ring 23 zur Anlage kommt. Wie auch der Figur 1 zu entnehmen ist, wird das Außengewinde des Zylinderblocks 22 mit einem Innengewinde 15 im unteren Bereich des Hohlkörpers 10 verschraubt. Diese Schraubverbindung mitsamt dem O-Ring ist derart, dass sie keinen Dampf in den Messraum 12 der Messkammer 8 eindringen lässt. Lediglich durch eine waagerecht verlaufende Durchgangsöffnung 18, insbesondere eine Bohrung mit kleinem Durchmesser, kann Dampf vom Sterilisationsraum S in den Messraum 12 gelangen.

Wie in der entsprechenden DIN vorgeschrieben ist, kann anhand der Temperaturdifferenz und der Zeitverläufe der Temperaturen, die von den Temperatursensoren 3, 5 gemessen werden, auf die Effektivität des Sterilisationsprozesses in dem Autoklaven, in dem die Messvorrichtung 1 eingebracht ist, rückgeschlossen werden.

Wie der Figur 3 weiterhin zu entnehmen ist, weist die Halterung 20 eine zentrale Durchgangsöffnung 24 auf, durch die der zweite Temperatursensor 5 führbar ist. Die Halterung 20 selbst ist mittels eines unterseitig vorgesehenen Außengewindes 25 auf eine fest mit dem Grundkörper 2 verbundene Fassung 35 mit Innengewinde aufschraubbar. Diese Schraubverbindung, die insbesondere als Luerlock-Verbindung ausgebildet sein kann, weist ebenfalls die zentrale Durchgangsöffnung 24 auf.

Der Hohlkörper 10 ist aus einem Hochtemperaturkunststoff gefertigt, beispielsweise aus PEEK oder PTFE. Diese Kunststoffe halten auch hohen Temperaturen, wie sie in einem Dampfsterilisationsprozess auftreten, stand. Zudem ist ihre leichte Reinigung, z.B. von Kondensationswasser, möglich. Auch die Halterung 20 besteht vorzugsweise aus einem derartigen Kunststoff. Alternativ kann auch ein Metall für die Halterung 20 gewählt werden.

Durch die erfindungsgemäße Ausgestaltung lässt sich der Hohlkörper 10 der Messkammer 8 in einfacher Weise reversibel von der Halterung 20 lösen, um insbesondere die Innenwände des Hohlkörpers 10 mit einem Tuch leicht zu säubern. Auch kann beispielsweise bei entsprechender lösbarer Ausgestaltung der zweite Temperatursensor 5 bei Schadhaftigkeit ausgetauscht werden. Ähnliches gilt für die Halterung 20, die lösbar mit dem Grundkörper 2 verschraubt ist und deren zur Hohlkammer gerichtete Oberseite sich nunmehr leicht säubern lässt. Insgesamt ergibt sich eine Ausgestaltung der erfindungsgemäßen Messvorrichtung, die wesentlich einfacher bzw. leichter handhabbarer ist als die bekannten Messvorrichtungen.

## Patentansprüche

1. Messvorrichtung (1) zum Überprüfen der Effektivität eines in einem Autoklaven durchgeführten Dampfsterilisationsprozesses zumindest mittels Temperaturmessungen,
mit einem Grundkörper (2),
mit einem mit dem Grundkörper (2) in Verbindung stehenden ersten Temperatursensor (3) zum Messen der Temperatur im Sterilisationsraum (S) des Autoklaven,
mit einem mit dem Grundkörper (2) in Verbindung stehenden zweiten Temperatursensor (5) zum Messen der Temperatur in einem Messraum (12) eines zumindest einseitig offenen Hohlkörpers (10) einer gegenüber dem Sterilisationsraum (S) weitgehend abgeschirmten, mit dem Grundkörper (2) in Verbindung stehenden Messkammer (8), wobei ein Luftaustausch zwischen dem Sterilisationsraum (S) und dem Messraum (12) möglich ist,
mit einer in dem Grundkörper (2) untergebrachten Elektronik (40, 42) zum Speichern, Auswerten und/oder Weiterleiten der Temperatursignale,
wobei die Messkammer (8) den Hohlkörper (10) sowie eine am Grundkörper angeordnete Halterung (20) umfasst, wobei der Hohlkörper (10) über den zweiten Temperatursensor (5) gestülpt und von der Halterung (20) reversibel lösbar ist, und wobei beim Abnehmen des Hohlkörpers (10) der zweite Temperatursensor (5) frei zugänglich ist,
**dadurch gekennzeichnet, dass** die Halterung (20) den Messraum (12) der Messkammer (8) von dem Innenraum des Grundkörpers (2), in dem die Elektronik (40, 42) angeordnet ist, trennt.

2. Messvorrichtung nach Anspruch 1, **dadurch gekennzeichnet, dass** der Hohlkörper (10) hohlzylinderförmig ausgebildet ist.

3. Messvorrichtung nach Anspruch 1 oder 2, **dadurch gekennzeichnet, dass** der Hohlkörper (10) aus einem hochtemperaturfesten Kunststoff besteht, beispielsweise aus PEEK oder PTFE.

4. Messvorrichtung nach einem der Ansprüche 1 bis 3, **dadurch gekennzeichnet, dass** in dem Hohlkörper (10) mindestens eine Durchgangsöffnung (18) vorgesehen ist, welche den Sterilisationsraum (S) mit dem Inneren (12) der Messkammer (8) verbindet.

5. Messvorrichtung nach einem der vorhergehenden Ansprüche, **dadurch gekennzeichnet, dass** der Hohlkörper (10) zumindest teilweise die Halterung (20) übergreift.

6. Messvorrichtung nach einem der vorhergehenden Ansprüche, **dadurch gekennzeichnet, dass** die Halterung (20) mit dem Grundkörper (2) verschraubbar ist.

7. Messvorrichtung nach einem der vorhergehenden Ansprüche, **dadurch gekennzeichnet, dass** der Hohlkörper (10) mit der Halterung (20) verrastbar ist.

8. Messvorrichtung nach einem der vorhergehenden Ansprüche, **dadurch gekennzeichnet, dass** der zweite Temperatursensor (5) mit dem Grundkörper (2) reversibel verbindbar ist.

9. Messvorrichtung nach Anspruch 8, **dadurch gekennzeichnet, dass** der zweite Temperatursensor (5) durch die Halterung (20) hindurch zum Grundkörper (10) führbar ist.

10. Messvorrichtung nach einem der vorhergehenden Ansprüche, **g****ekennzeichnet durch** mindestens einen Drucksensor (30).

11. Messvorrichtung nach Anspruch 10, **dadurch gekennzeichnet, dass** der mindestens eine Drucksensor (30) mit dem Grundkörper (2) verbindbar ist.

12. Messvorrichtung nach einem der vorhergehenden Ansprüche, **g****ekennzeichnet durch** einen elektronischen Speicher (40), sog. Datenlogger, in dem Grundkörper (2), wobei der Speicher (40) zum Abspeichern der gemessenen Temperaturwerte und ggf. der Druckwerte ausgebildet ist.

13. Messvorrichtung nach Anspruch 12, **dadurch gekennzeichnet, dass** der elektronische Speicher (40) durch drahtlose Datenübertragung (42, 43) auslesbar ist.

## Claims

1. A measuring device (1) for checking the effectiveness of a steam sterilization process performed in an autoclave, at least by means of temperature measurements,
having a base body (2),
having a first temperature sensor (3) connected to the base body (2) for measuring the temperature in the sterilization chamber (S) of the autoclave,
having a second temperature sensor (5) connected to the base body (2) for measuring the temperature in a measurement space (12) of a
hollow body (10), open on at least one side, of a measurement chamber (8) connected to the base body (2) and largely shielded from the sterilization chamber (S), wherein an air exchange between the sterilization chamber (S) and the measurement chamber (12) is possible,
having electronics (40, 42) disposed in the base body (2) for storing, evaluating, and/or relaying the temperature signals,
wherein the measurement chamber (8) comprises the hollow body (10) and a mounting element (20) disposed on the base body, the hollow body (10) is placed over the second temperature sensor (5) and is reversibly removable from the mounting element (20), and wherein the second temperature sensor (5) is freely accessible when the hollow body (10) is removed, **characterized in that** the mounting element (20) separates the measurement space (12) of the measurement chamber (8) from the interior of the base body (2), in which the electronics (40, 42) are disposed.

2. The measuring device according to claim 1, **characterized in that** the hollow body (10) is designed as a hollow cylinder.

3. The measuring device according to claim 1 or 2, **characterized in that** the hollow body (10) is made of a highly temperature-resistant plastic, such as PEEK or PTFE.

4. The measuring device according to one of the claims 1 through 3, **characterized in that** at least one through-hole (18) in the hollow body (10) is provided that connects the sterilization chamber (S) to the interior (12) of the measurement chamber (8).

5. The measuring device according to one of the preceding claims, **characterized in that** the hollow body (10) at least partially overlaps the mounting element (20).

6. The measuring device according to one of the preceding claims, **characterized in that** the mounting element (20) can be screwed to the base body (2).

7. The measuring device according to one of the preceding claims, **characterized in that** the hollow body (10) can be locked to the mounting element (20).

8. The measuring device according to one of the preceding claims, **characterized in that** the second temperature sensor (5) can be reversibly connected to the base body (2).

9. The measuring device according to claim 8, **characterized in that** the second temperature sensor (5) can be fed through the mounting element (20) to the base body (10).

10. The measuring device according to one of the preceding claims, **characterized by** at least one pressure sensor (30).

11. The measuring device according to claim 10, **characterized in that** the at least one pressure sensor (30) can be connected to the base body (2).

12. The measuring device according to one of the preceding claims, **characterized by** an electronic memory (40), known as a data logger, in the base body (2), wherein the memory (40) is designed for storing the measured temperature values and optionally the pressure values.

13. The measuring device according to claim 12, **characterized in that** the electronic memory (40) can be read out by wireless data transfer (42, 43).

## Revendications

1. Dispositif de mesure (1) pour le contrôle de l'efficience d'un processus de stérilisation à la vapeur effectué dans un autoclave au moyen au moins de mesures de température,
avec un corps de base (2),
avec un premier capteur de température (3) en relation avec ledit corps de base (2) pour la mesure de la température dans la chambre de stérilisation (S) de l'autoclave,
avec un second capteur de température (5) en relation avec ledit corps de base (2) pour la mesure de la température dans un espace de mesure (12) d'un corps creux (10) ouvert au moins sur un côté d'une chambre de mesure (8) quasiment abritée de la chambre de stérilisation (S) et en relation avec ledit corps de base (2), sachant qu'un échange d'air est possible entre la chambre de stérilisation (S) et la chambre de mesure (12),
avec un composant électronique (40, 42) logé dans le corps de base (2) pour mémoriser, analyser et/ou retransmettre les signaux de température,
sachant que la chambre de mesure (8) comprend le corps creux (10) ainsi qu'un support (20) disposé au corps de base, que ledit corps creux (10) est retourné sur le second capteur de température (5) et, d'une façon réversible, détachable du support (20), et que le second capteur de température (5) est librement accessible après l'enlèvement du corps creux (10), **caractérisé en ce que** le support (20) sépare l'espace de mesure (12) de la chambre de mesure (8) de l'espace intérieur du corps de base (2), dans lequel est disposé le composant électronique (40, 42).

2. Dispositif de mesure selon la revendication 1, **caractérisé en ce que** le corps creux (10) se présente sous la forme d'un cylindre creux.

3. Dispositif de mesure selon l'une quelconque des revendications 1 ou 2, **caractérisé en ce que** le corps creux (10) se compose d'une matière synthétique résistante aux hautes températures, par exemple de PEEK ou de PTFE.

4. Dispositif de mesure selon l'une quelconque des revendications 1 à 3, **caractérisé en ce qu'**il est prévu, dans le corps de base (10), au moins un orifice de passage (18) qui relie la chambre de stérilisation (S) avec l'intérieur (12) de la chambre de mesure (8).

5. Dispositif de mesure selon l'une quelconque des revendications précédentes, **caractérisé en ce que** le corps creux (10) recouvre au moins partiellement le support (20).

6. Dispositif de mesure selon l'une quelconque des revendications précédentes, **caractérisé en ce que** le support (20) peut être vissé au corps de base (2).

7. Dispositif de mesure selon l'une quelconque des revendications précédentes, **caractérisé en ce que** le corps creux (10) peut s'enclencher dans le support (20).

8. Dispositif de mesure selon l'une quelconque des revendications précédentes, **caractérisé en ce que** le second capteur de température (5) peut être relié de manière réversible au corps de base (2).

9. Dispositif de mesure selon la revendication 8, **caractérisé en ce que** le second capteur de température (5) peut être conduit au corps de base (10) à travers le support (20).

10. Dispositif de mesure selon l'une quelconque des revendications précédentes, **caractérisé par** au moins un capteur de pression (30).

11. Dispositif de mesure selon la revendication 10, **caractérisé en ce que** l'au moins un capteur de pression (30) peut être relié au corps de base (2).

12. Dispositif de mesure selon l'une quelconque des revendications précédentes, **caractérisé par** une mémoire électronique (40), appelée enregistreur de données, dans le corps de base (2), sachant que ladite mémoire (40) se présente sous une forme adaptée pour mémoriser les valeurs de température mesurées et, le cas échéant, les valeurs de pression mesurées.

13. Dispositif de mesure selon la revendication 12, **caractérisé en ce que** la mémoire électronique (40) peut être lue par transmission sans fil des données (42, 43).
